Europäisches Patentamt

European Patent Office

Office européen des brevets

(19)

(11) Numéro de publication: **0 330 588**
**A1**

(12)

# DEMANDE DE BREVET EUROPEEN

(21) Numéro de dépôt: 89420051.8

(22) Date de dépôt: 15.02.89

(51) Int. Cl.⁴: **A 61 F 13/06**

(30) Priorité: 15.02.88 FR 8802172

(43) Date de publication de la demande:
30.08.89 Bulletin 89/35

(84) Etats contractants désignés:
AT BE CH DE ES GB GR IT LI LU NL SE

(71) Demandeur: PICHON FRERES Société Anonyme
2, rue Cussinel
F-42000 St Etienne (Loire) (FR)

(72) Inventeur: Pichon, Jean Claude
8, allée Jean Guitton
F-42000 Saint Etienne Loire (FR)

(74) Mandataire: Perrier, Jean-Pierre et al
Cabinet GERMAIN & MAUREAU 12 rue de la République
F-42000 St-Etienne (FR)

(54) **Chevillère de maintien.**

(57) Chevillère de maintien du type composé d'une chaussette extensible (2) à talon ouvert (3) et de moyens (4) de blocage de la cheville.

Selon l'invention, ces moyens de blocage sont constitués par deux boucles (5a-5b) qui, formées dans une bande extensible et aptes à s'accrocher sur le talon, en extension et sans plissage, sont fixées, chacune et par leurs extrémités sur la partie de la chaussette extensible recouvrant le coup de pied et de part et d'autre du plan médian vertical de la chaussette.

FIG.1

EP 0 330 588 A1

## Description

## Chevillère de maintien.

Pour la pratique de certains sports, il est connu de renforcer la cheville au moyen d'une chevillère de maintien. Celle-ci réduit les risques de torsion de la cheville vers l'intérieur, en varus, et vers l'extérieur, en valgus.

Traditionnellement, de telles chevillères sont constituées par une chaussette en tissu élastique qui freine les mouvements de torsion. Il s'avère, à l'usage, que ce freinage avec rappel élastique est insuffisant et ne peut pas être amélioré en augmentant la tension donnée au tissu élastique car, alors, cette tension, répartie sur toute la cheville mais aussi sur le bas de la jambe et le pied, pourrait perturber la circulation sanguine.

On connait aussi des chevillères, du type décrit dans le brevet U.S. N° 2 645 222, qui sont composées d'une bande élastique enroulée en tension autour de la cheville. Cette solution améliore le maintien mais crée des surépaisseurs qui, soit s'opposent au port de la chaussure, soit blessent le porteur en créant avec le serrage de la chaussure, des micro hématomes localisés.

La présente invention a pour but de fournir une chevillère de maintien permettant d'obtenir un meilleur blocage élastique de la cheville sans pour autant présenter des inconvénients lors de son port avec chaussure.

Dans cette chevillère, du type composé d'une chaussette extensible avec moyens de blocage de la cheville, les moyens de blocage sont constitués par deux boucles qui, formées dans une bande extensible et aptes à s'accrocher sur le talon, en extension et sans plissage, sont fixées, chacune et par leurs extrémités, sur la partie de la chaussette extensible recouvrant le coup de pied et de part et d'autre du plan médian vertical de la chaussette.

Ainsi, les deux boucles étirées de part et d'autre de la cheville et plaquées sur des zones localisées, peuvent fournir une tension élastique bien supérieure à celle d'une chevillère traditionnelle, constituée par une seule chaussette élastique, et assurer ainsi un meilleur maintien. De plus, de par leur structure, la mise en place des boucles sur le talon est immédiate, fournit une tension constante et ne procure aucune gêne au port de chaussure.

Dans une forme d'exécution, les coutures de fixation des boucles assurent aussi la fixation d'une nappe formant une poche apte à recevoir un coussin amortisseur.

Lorsque la poche centrale reçoit un coussin, ce dernier amorti les coups que le sportif reçoit sur le coup de pied et procure ainsi un rôle protecteur apprécié.

D'autres caractéristiques et avantages ressortiront de la description qui suit en référence au dessin schématique annexé représentant, à titre d'exemples non limitatifs, une forme d'exécution de cette chevillère.

Figure 1 est une vue en perspective de la chevillère au repos,

Figure 2 est une vue en perspective du coussin,

Figures 3 et 4 sont des vues en perspective montrant la chevillère lorsqu' elle est mise en place sur un pied droit et sur un pied gauche et vue respectivement côté extérieur et côté intérieur de la jambe.

Cette chevillère est donc composée d'une chaussette extensible 2 à talon ouvert 3 et comporte des moyens 4 d'accrochage sur le talon.

Selon l'invention, ces moyens d'accrochage sont constitués par deux boucles 5a et 5b réalisés dans des bandes extensibles. Chacune des boucles est fixée par ses extrémités au niveau de la partie de la chaussette venant sur le coup de pied, de part et d'autre du plan médian P de cette chaussette et suivant des coutures 6 formant un V dont la pointe est dirigée sur l'extérieur.

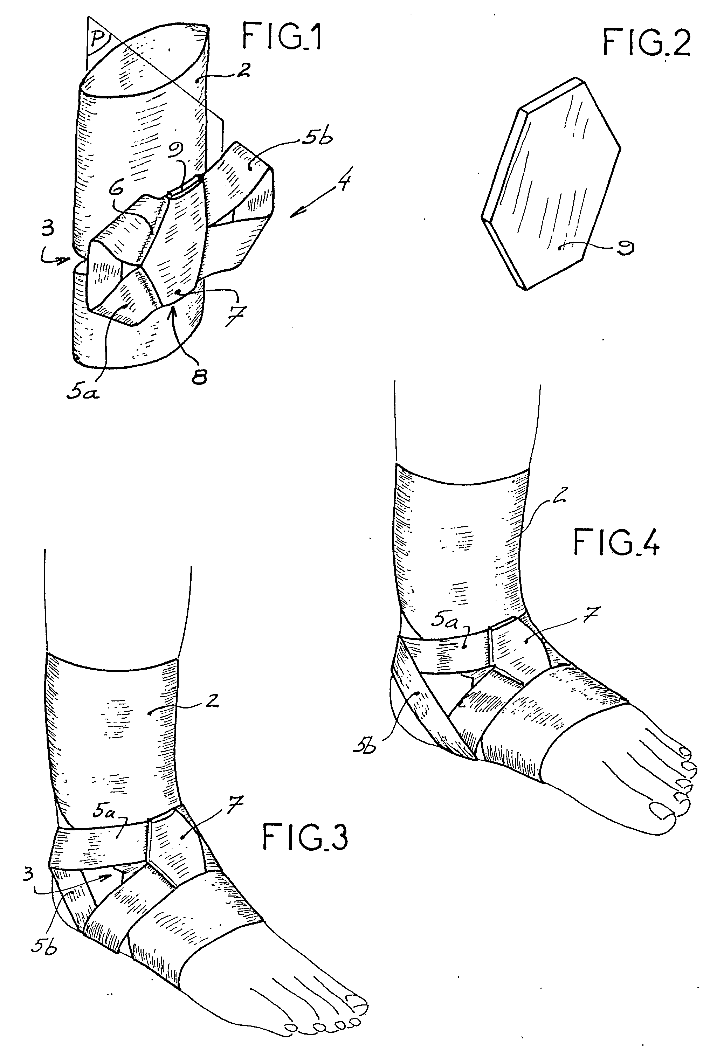

Dans une forme d'exécution, ces coutures 6 sont aussi utilisées pour fixer sur le coup de pied, une bande extensible 7, constituant une poche 8 pouvant recevoir un rembourrage élastique 9 en mousse ou autre. La figure 2 montre que ce rembourrage a une forme de losange tronqué verticalement.

En raison de sa structure, cette chevillère peut être passée sur le pied droit ou sur le pied gauche, comme le montre les figures 3 et 4. Après que la chaussette ait été engagée sur le pied, chacune des deux boucles 5a-5b est étirée latéralement et accrochée sous le talon en extension et sans pli. Les figures 3 et 4 montrent que la superposition des bandes constitutives des boucles s'effectue dans la zone du talon ouvert et ne crée donc pas de surépaisseur excessive gênante avec le port d'une chaussure.

Lorsque la chevillère est en place, ses deux boucles tendues renforcent les effets des ligaments latéraux, respectivement antérieur et extérieur, améliorent le maintien de la mortaise tibio-péronière sur le tenon astragalien, emprisonnent l'astragale et le calcanéum et assurent la stabilisation de la cheville.

La chevillère peut être portée avec ou sans le coussin de rembourrage. Lorsqu' elle comporte ce coussin, ce dernier amorti les chocs sur le coup de pied et assure ainsi un rôle de protection très apprécié.

## Revendications

1. Chevillère de maintien du type composé d'une chaussette extensible 2 à talon ouvert 3 et de moyens 4 de blocage de la cheville, caractérisée en ce que ces moyens sont constitués par deux boucles 5a-5b qui, formées dans une bande extensible et aptes à s'accrocher sur le talon, en extension et sans plissage, sont fixées, chacune et par leurs extrémités sur la partie de la chaussette extensible recouvrant le coup de pied et de part et d'autre du plan médian vertical de la chaussette.

2. Chevillère selon la revendication 1 caractérisée en ce que les extémités de chaque boucles 5a-5b sont fixées sur la chaussette 2 par des coutures 6 formant un V.

3. Chevillère selon l'ensemble des revendications 1 et 2 caractérisée en ce que les coutures 6 de fixation des boucles 5a-5b assurent aussi la fixation d'une nappe 7 formant une poche 8 apte à recevoir un coussin amortisseur 9.

4. Chevillère selon la revendication 3 caractérisée en ce que le coussin amortisseur 9 présente la forme d'un losange tronqué verticalement.

FIG.1

FIG.2

FIG.3

FIG.4

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | US-A-4 367 733  (STROMGREN) <br> * figures 1,3 * <br> --- | 1 | A 61 F   13/06 |
| A | EP-A-0 114 560  (BERTHEAS) <br> * revendication 1; figure 1 * <br> --- | 1 | |
| A | US-A-4 693 241  (TRZNADEL) <br> * figures * <br> --- | 1 | |
| D,A | US-A-2 645 222  (CAPOSSELA) <br> * figures 1-3 * <br> --- | 1 | |
| P,A | EP-A-0 286 554  (ETS PICHON FRERES) <br> * revendication 1; figures 1,2 * <br> ----- | 1 | |

DOMAINES TECHNIQUES
RECHERCHES (Int. Cl.4)

A 61 F   13/00

Le présent rapport a été établi pour toutes les revendications

| Lieu de la recherche | Date d'achèvement de la recherche | Examinateur |
|---|---|---|
| BERLIN | 17-05-1989 | KANAL P K |